# EUROPEAN PATENT SPECIFICATION

(11) **EP 3 091 068 B1**
(45) Date of publication and mention of the grant of the patent: **09.01.2019**
(21) Application number: 14877171.0
(22) Date of filing: 31.12.2014
(51) Int. Cl.: C12M 1/38, C12M 1/36, C12M 1/34

(54) **FULL-AUTOMATIC MICROBIAL DETECTION AND ENRICHMENT SYSTEM AND ENRICHMENT METHOD THEREOF**
VOLLAUTOMATISCHE MIKROBENDETEKTION UND ANREICHERUNGSSYSTEM UND ANREICHERUNGSVERFAHREN DAFÜR
SYSTÈME ENTIÈREMENT AUTOMATIQUE DE DÉTECTION ET D'ENRICHISSEMENT MICROBIEN, ET SON PROCÉDÉ D'ENRICHISSEMENT

(30) Priority: 31.12.2013 CN 201310751864
(43) Date of publication of application: 09.11.2016
(73) Proprietor: Niu, Gang, Beijing 100009 (CN); Wang, Huashan, Tianjin 300222 (CN)
(72) Inventor: NIU, Gang, Dongcheng District Beijing 100009 (CN)
(74) Representative: Delumeau, François Guy
(86) International application number: PCT/CN2014/095848
(87) International publication number: WO 2015/101326

(56) References cited:
- WO-A1-99/23243
- CN-A- 1 285 878
- CN-U- 201 756 550
- CN-U- 203 833 938
- US-A1- 2004 009 572
- DATABASE WPI Week 201159 Thomson Scientific, London, GB; AN 2011-J67985 XP002773392, -& CN 201 883 098 U (WANG H) 29 June 2011 (2011-06-29)
- DATABASE WPI Week 201179 Thomson Scientific, London, GB; AN 2011-M47760 XP002773393, -& CN 102 174 395 A (GUANGZHOU BIOLOGICAL MEDICINE & HEALTH R) 7 September 2011 (2011-09-07)

## Description

### TECHNICAL FIELD

The present invention relates to the technical field of microorganism detection. More particularly, the invention relates to a full-automatic microorganism detecting enrichment system and the enrichment method thereof for implementing the enrichment process in the detection of microorganisms.

### BACKGROUND ART

At present, all microorganism detecting enrichments almost are conducted manually. The units for sterilizing, bacteria enrichment driving, filling the culture medium and positive bacteria filling are independent of each other, and the environments for filling positive bacteria and collecting bacteria are different, which means the whole process needs to be conducted in the different environments, and transmitting the to-be-detected samples between different environments has to rely on manual operation, thus it is easy to be influenced by human factors, which might prone to produce false positive or false negative results and affect the accuracy and timeliness of detection.

Document WO 99/23243 A1 discloses a method and an apparatus for concentrating and detecting microorganisms in a culture medium using a syringe system for aspirating the microorganism connected to a gas conduit, a gas or gas mixture being directed from a compressed bottle into the syringe.

With the increase of people's requirements for food and drug safety, a full-automatic microorganism enrichment system without human intervention is an inevitable demand for food and drug safety.

### CONTENTS OF INVENTION

The aim of the present invention is providing a full-automatic microorganism detecting enrichment system and the enrichment method thereof, which can realize an automated enrichment of the to-be-detected samples, effectively avoid false positive or false negative results caused by an effect of human factors, and achieve an accurate detection result.

To reach above aims, the present invention employs following solutions.

A full-automatic microorganism detecting enrichment system comprises:
a preset operating position, for placing to-be-detected samples, a culture medium containing culture, a pipeline filter specially for collecting bacteria and a filter head; the filter utilizing a filter film for filtering the to-be-detected samples and enriching the microorganisms possibly contained in the to-be-detected samples;
a sterilization cabin for conducting sterilization, a middle package removing cabin for removing the middle package, an inner package removing cabin for removing the inner package, an enrichment operating cabin for injecting the to-be-detected samples into a filter to filter the enriched microorganism, enclosing the filter bottom by a filter head, and selectively injecting the culture into the filter, a buffer cabin for cutting the filter pipeline after the enrichment, and a positive bacteria filling cabin for filling the positive bacteria, being disposed adjacent successively behind the preset operating position; above said cabins being connected and separated respectively by a cabin separating mechanism;
a transmitting apparatus, for transmitting the to-be-detected samples placed on the preset operating position, the culture medium, the filter and the filter head into each corresponding cabin respectively, the transmitting apparatus starting from the preset operating position and running through above said cabins; and
a industrial control computer, for electrically connecting above each cabin, each cabin separating mechanism and the transmitting apparatus.

In a preferred embodiment, a fixing device is provided on the transmitting apparatus for fixing the to-be-detected samples, the culture medium containing the culture, the filter and the filter head, and/or
the transmitting apparatus could be divided into a to-be-detected sample transmitting apparatus, a culture medium transmitting apparatus, a filter transmitting apparatus and a filter head transmitting apparatus; and/or
position sensors are provided on the preset positions of the transmitting apparatus within each cabin and electrically connected with the industrial control computer; and/or
the transmitting apparatus is a belt conveyor or a roller transmitting apparatus; the belt or roller could be driven by rotating magnetic fluid within each cabin actuated by a motor outside each cabin, or directly driven by a motor provided inside of each cabin; and/or
each cabin separating mechanism is a separating valve.

In a preferred embodiment, an air pressure regulating device and a ventilation device are provided outside of each cabin to adjust the cabin pressure; each air pressure regulating device and each ventilation device electrically connect to said industrial control computer respectively; each air pressure regulating device contains a pressure sensor for detecting the pressure of each cabin, and each pressure sensor electrically connects to the industrial control computer; and/or
sterilization devices are respectively arranged outside said sterilization cabin, the enrichment operating cabin, and the buffer cabin for sterilizing corresponding cabins.

In a preferred embodiment, a middle package removing device and an inner package removing device, which are used for removing the middle package and inner package of the filter and the filter head, are respectively arranged above said transmitting apparatus within said middle package removing cabin and said inner package removing cabin; the inner package removing device and the middle package removing device are electrically connected with the industrial control computer respectively.

In a preferred embodiment, the pipeline filters are three parallel tanks with outlets on both ends, each tank has filter film on the bottom, after being filled with the to-be-detected samples, microorganisms are enriched by the filter film; the pipelines of filter are three parallel pipelines, one end of which is provided on the top of said filter and communicated with each tank, and the other end of which is a filter needle; said filter has a set position in the enrichment operating cabin, and the set position is where the bottom of each tank locates in the supporting hole of the waste liquid trough;
an enrichment operation manipulator is arranged above said transmitting apparatus in the enrichment operating cabin, the enrichment operation manipulator inserts the filter needle into the to-be-detected samples and replaces the filter needle with a culture needle, then inserting it into the culture of the culture medium; the enrichment operation manipulator can make the filter shift between the set position and the transmitting apparatus; an enrichment driving mechanism is provided on one side of the enrichment operation manipulator in the proximity of said buffer cabin for driving the to-be-detected items or culture to be filled into three parallel pipelines and to enter into said filter;
a packaging mechanism is provided near said set position in the enrichment operating cabin for packaging the bottom of each tank by the filter head after the process of filtering and enriching;
the enrichment operation manipulator, the enrichment driving mechanism and the packaging mechanism are electrically connected to the industrial control computer respectively.

In a preferred embodiment, said enrichment driving mechanism is a peristaltic pump, a first bracket and a second bracket are provided on the pump head of the peristaltic pump for clamping the three parallel pipelines when the enrichment operation manipulator holds the filter needle to insert into the to-be-detected samples, the first bracket is retractably fixed on one side of the peristaltic pump head near the enrichment operation manipulator, the second bracket is provided on the pump head of the peristaltic pump retractably and rotatablely; the second bracket has a first position and a second position, on the first position, the second bracket is arranged side by side at the side of the first bracket for clamping the three parallel pipelines together with the first bracket, on the second position, the second bracket rotates to a location where the three parallel pipelines can be correspondingly placed into the pump head of the peristaltic pump; when operating, the first bracket and the second bracket clamp the three parallel pipelines respectively, then the second bracket is rotated to the second position and the first and second brackets retract back, thereby putting the three parallel pipelines into the pump head of the peristaltic pump
said enrichment operation manipulator further includes a culture medium heat-seal mechanism for sealing at most two pipelines of the three parallel pipelines before the culture medium entering into said filter after that said filter finished filtering and enriching the to-be-detected samples, thereby the culture medium could be selectively injected into the microorganism enriched filters;
a triple pressure sensor is provided in the enrichment operating cabin to detect the inner pressure of said filter;
the peristaltic pump, the first bracket, the second bracket, the culture medium heat-seal mechanism and the triplet pressure sensor are electrically connected with the industrial control computer, respectively.
In a preferred embodiment, a pipeline cutting mechanism are provided above the transmitting apparatus in said buffer cabin, and
the pipeline cutting mechanism is electrically connected with the industrial control computer.

In a preferred embodiment, said positive bacteria filling cabin has a heating function, a positive bacteria filling mechanism is provided above the transmitting apparatus in the positive bacteria filling cabin; a temperature control apparatus is further provided in the positive bacteria filling cabin;
the positive bacteria filling mechanism and the temperature control apparatus are electrically connected with the industrial control computer, respectively; and/or
the industrial control computer is a form of the host computer, and each cabin has separate control unit.

A detection method applied to any above full-automatic microorganism detecting enrichment system, comprises following steps of:
the first step: placing to-be-detected samples, the culture medium containing culture, the pipeline filter specially for collecting bacteria and the filter head on the transmitting apparatus of the preset operating position;
the second step: opening the cabin separating mechanism between the preset operating position and the sterilization cabin, transmitting the to-be-detected samples, the culture medium containing culture, the pipeline filter specially for collecting bacteria and the filter head into said sterilization cabin, closing the cabin separating mechanisms on both sides of the sterilization cabin and carrying out sterilization, then ventilating for balancing the pressure in the sterilization cabin after finishing the sterilization;
the third step: opening the cabin separating mechanism between the sterilization cabin and the middle package removing cabin, transmitting the to-be-detected samples, the culture medium containing culture, the pipeline filter specially for collecting bacteria and the filter head into the middle package removing cabin, closing the cabin separating mechanisms on both sides of the middle package removing cabin, and removing the middle packages of the filter and the filter head;
the fourth step: opening the cabin separating mechanism between the middle package removing cabin and the inner package removing cabin, transmitting the to-be-detected samples, the culture medium containing culture, the pipeline filter specially for collecting bacteria and the filter head into the inner package removing cabin, closing the cabin separating mechanisms on both sides of inner package removing cabin, and removing the inner package of the filter and the filter head;
the fifth step: opening the cabin separating mechanism between the inner package removing cabin and the enrichment operating cabin, transmitting the to-be-detected samples, the culture medium containing culture, the pipeline filter specially for collecting bacteria and the filter head into the enrichment operating cabin, closing the cabin separating mechanisms on both sides of the enrichment operating cabin, filtering and enriching the microorganisms contained in the to-be-detected samples in the filter, enclosing the filter bottom by the filter head, then selectively injecting the culture into the filter, placing the enriched and enclosed filter on the transmitting apparatus, obtaining appropriate number of the enriched and enclosed filters;
the sixth step: opening the cabin separating mechanism between the enrichment operating cabin and the buffer cabin, transmitting the enriched and enclosed filters into the buffer cabin, closing the cabin separating mechanisms on both sides of buffer cabin, cutting the pipeline of enriched and enclosed filters; and
the seventh step, opening the cabin separating mechanism between the buffer cabin and the positive bacteria filling cabin, transmitting the enriched and enclosed filters into the positive bacteria filling cabin, closing the cabin separating mechanisms on both sides of the positive bacteria filling cabin, filling the positive bacteria into the enriched and enclosed filters after finishing the pipeline cutting, then completing the microorganism enrichment of the detected samples.

In a preferred embodiment, each time the cabin separating mechanism between the preset operating position, the sterilization cabin, the middle package removing cabin, the inner package removing cabin and the enrichment operating cabin open, the pressure of the former cabin is lower than that of the latter cabin, to guarantee no bacteria being taken into the enrichment operating cabin; and each time the cabin separating mechanisms between the enrichment operating cabin, the buffer cabin and the positive bacteria filling cabin open, the pressure of the former cabin is higher than that of the latter cabin, to guarantee no positive bacteria being adversely transferred into the enrichment operating cabin; and/or
in said seventh step, after closing the cabin separating mechanisms on both sides of the positive bacteria filling cabin, a further step is included: sterilizing the buffer cabin; and/or
said filters are three parallel tanks with outlets on both ends, the culture are selectively injected into at most two of those tanks; and/or
in said fifth step, used samples and culture medium are transferred back to the inner package removing cabin by the transmitting apparatus for recycling.

The beneficial effects of the present invention are as follows. The present invention provides a full-automatic microorganism detecting enrichment system and the enrichment method, which can achieve a fully automated enrichment of the microorganism in the to-be-detected samples, needs no manual operation, and saves time and labor force. Further, it can effectively avoid false positive or false negative result caused by an effect of human factors, and achieve sterile and automatic operation with an accurate detecting result. The enrichment process is less error-prone and is able to be operated continuously, with no need to pause and sterile the enrichment operating cabin after stopping the enrichment operation, consequently it has a better implementation effect.

### DESCRIPTION OF DRAWINGS

Figure 1 is a schematic top view for a preferred embodiment of a full-automatic microorganism detecting enrichment system in the present invention.
Figure 2 is a schematic main view for a preferred embodiment of a full-automatic microorganism detecting enrichment system in the present invention.
Figure 3 is a flow chart for a preferred embodiment of a full-automatic microorganism detecting enrichment system in the present invention.

### Reference Signs in Figures:

- 101: preset operation position
- 102: sterilization cabin
- 103: middle package removing cabin
- 104: inner package removing cabin
- 105: enrichment operating cabin
- 106: buffer cabin
- 107: positive bacteria filling cabin
- 110: air pressure regulating device
- 120: sterilization device
- 130: ventilation device
- 200: position sensor
- 310: middle package removing device
- 320: inner package removing device
- 140: cabin separating mechanism
- 400: transmitting apparatus
- 500: enrichment driving mechanism
- 510: enrichment operation manipulator
- 520: pipeline cutting mechanism
- 530: packaging mechanism
- 700: positive bacteria filling mechanism
- 800: industrial control computer
- 900: triplet pressure sensor
- 1000: to-be-detected samples
- 1100: filter

### DESCRIPTION OF EMBODIMENTS

First of all, it needs to be declared that following description is only used for explaining the possible embodiments of the present invention, however, the description should not be considered as a limit to the protection scope of the present invention.

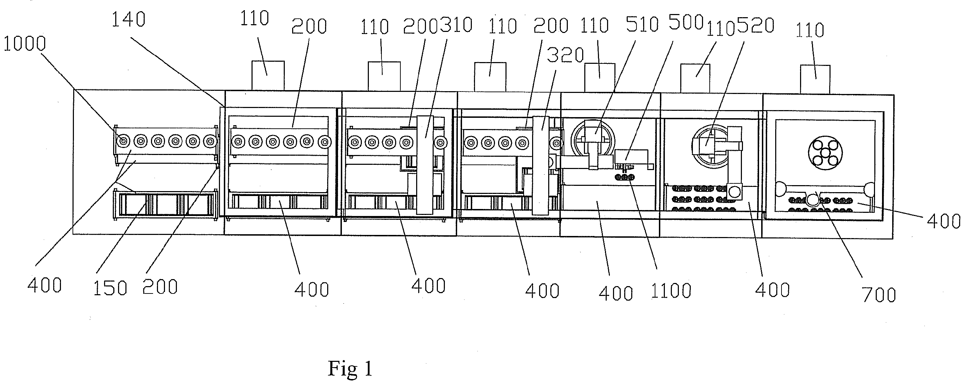

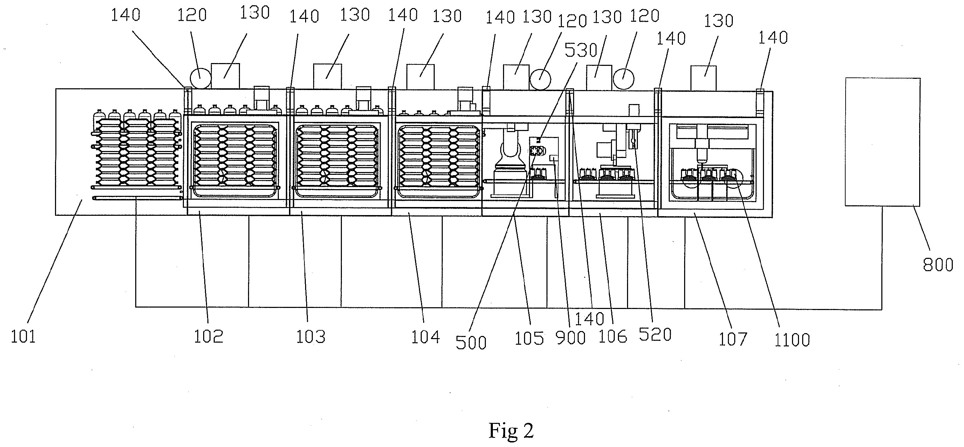

As shown in Figures 1 and 2, the present invention provides a full-automatic microorganism detecting enrichment system, comprising:
A preset operating position 101, behind which there are disposed adjacent successively by a sterilization cabin 102 for conducting sterilization, a middle package removing cabin 103 for removing the middle package, an inner package removing cabin 104 for removing the inner package, an enrichment operating cabin 105 for injecting the to-be-detected samples into a filter to filter the enriched microorganism, enclosing the filter bottom by a filter head, and selectively injecting the culture into the filter, a buffer cabin 106 for cutting the filter pipeline after the enrichment, and a positive bacteria filling cabin 107 for filling the positive bacteria; said cabins being connected and separated respectively by a cabin separating mechanism 140; a transmitting apparatus 400 and a industrial control computer 800.

The preset operating position 101 is used for placing to-be-detected samples 1000, a culture medium containing culture, a pipeline filter 1100 specially for collecting bacteria and a filter head. Said filter utilizes a filter film for filtering the to-be-detected samples and enriching the microorganisms possibly contained in the to-be-detected samples.

The transmitting apparatus 400 is used for transmitting the to-be-detected samples placed on the preset operating position 101, the culture medium, the filter 1100 and the filter head into said each corresponding cabin respectively. The transmitting apparatus 400 starts from the preset operating position and runs through above cabins.

The industrial control computer 800 is electrically connected with said cabins, each cabin separating mechanism 140 and the transmitting apparatus 400 to control said cabins performing corresponding actions.

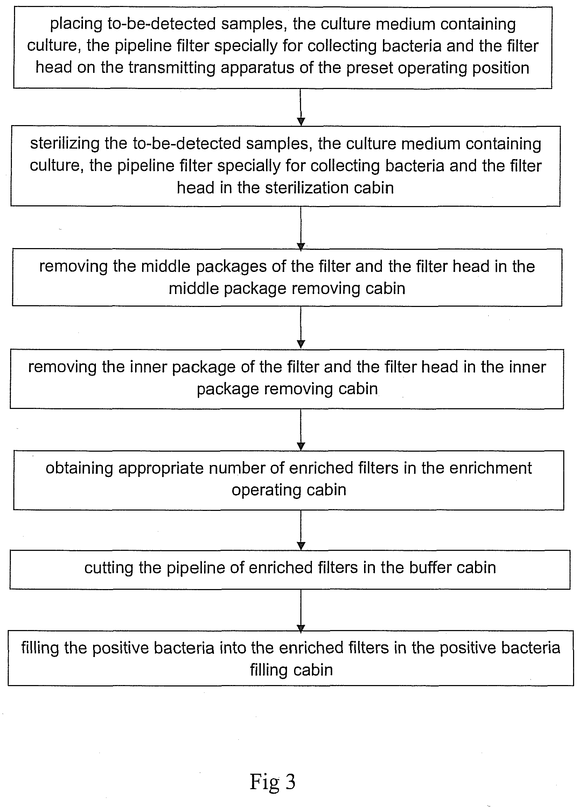

As shown in Figure 3, the present invention further provides a full-automatic microorganism enriching method, which comprises following steps of:
(i) first, placing to-be-detected samples 1000, the culture medium containing culture, the pipeline filter 1100 specially for collecting bacteria and the filter head on the transmitting apparatus 400 of the preset operating position;
(ii) second, opening the cabin separating mechanism 140 between the preset operating position 101 and the sterilization cabin 102, transmitting the to-be-detected samples 1000, the culture medium containing culture, the pipeline filter 1100 specially for collecting bacteria and the filter head into the sterilization cabin 102, closing the cabin separating mechanisms 140 on both sides of the sterilization cabin 102 and carrying out sterilization, then ventilating for balancing the pressure in the sterilization cabin 102 after finishing the sterilization;
(iii) third, opening the cabin separating mechanism between the sterilization cabin 102 and the middle package removing cabin 103, transmitting the to-be-detected samples 1000, the culture medium containing culture, the pipeline filter 1100 specially for collecting bacteria and the filter head into the middle package removing cabin 103, closing the cabin separating mechanisms 140 on both sides of the middle package removing cabin 103, and removing the middle packages of the filter 1100 and the filter head;
(iv) fourth, opening the cabin separating mechanism 140 between the middle package removing cabin 103 and the inner package removing cabin 104, transmitting the to-be-detected samples 1000, the culture medium containing culture, the pipeline filter 1100 specially for collecting bacteria and the filter head into the inner package removing cabin 104, closing the cabin separating mechanisms 140 on both sides of inner package removing cabin 104, and removing the inner package of the filter 1100 and the filter head;
(v) fifth, opening the cabin separating mechanism 140 between the inner package removing cabin 104 and the enrichment operating cabin 105, transmitting the to-be-detected samples 1000, the culture medium containing culture, the pipeline filter 1100 specially for collecting bacteria and the filter head into the enrichment operating cabin 105, closing the cabin separating mechanisms 140 on both sides of the enrichment operating cabin 105,filtering and enriching the microorganisms contained in the to-be-detected sampleslOOO in the filter 1100, enclosing the filter bottom by the filter head, then selectively injecting the culture into the filter 1100, placing the enriched and enclosed filter 1100 on the transmitting apparatus, obtaining appropriate number of the enriched and enclosed filters;
(vi) sixth, opening the cabin separating mechanism 140 between the enrichment operating cabin 105 and the buffer cabin 106, transmitting the enriched and enclosed filters 1100 into the buffer cabin 106, closing the cabin separating mechanisms 140 on both sides of buffer cabin 105, cutting the pipeline of enriched and enclosed filters 1100;
(vii) seventh, opening the cabin separating mechanism 140 between the buffer cabin 106 and the positive bacteria filling cabin 107, transmitting the enriched and enclosed filters 1100 into the positive bacteria filling cabin 107, closing the cabin separating mechanisms 140 on both sides of the positive bacteria filling cabin 107, filling the positive bacteria into the enriched and enclosed filters 1100 after finishing the pipeline cutting, then completing the microorganism enrichment of the detected samples.

To sum up, the present invention is suitable for the enrichment required by the microorganism limit test in the field of food and drug safety. The whole process is controlled by the industrial control computer 800, thus it achieves the automation and eliminates the false positive or false negative results possibly caused by human factors.

In a preferred embodiment of the present invention, as shown in figures 1 and 2, a fixing device 150 is provided on the transmitting apparatus 400 for fixing the to-be-detected samples 1000, the culture medium containing the culture, the filter 1100 and the filter head, in order to ensure that the samples 1000, the culture medium containing the culture, the filter 1100 and the filter head would not be misplaced or dumped in the process of conveying due to inertia, which will cause contaminations within each cabin and affect the accuracy of the operation in each cabin.

The transmitting apparatus 400 could be divided into a to-be-detected sample transmitting apparatus, a culture medium transmitting apparatus, a filter transmitting apparatus and a filter head transmitting apparatus, in order to transmit the corresponding items and lower the complexity of the transmitting apparatus 400, for example, it only needs to provide a filter transmitting apparatus in the buffer cabin 106 and positive bacteria filling cabin 107, and doesn't need other three type of transmitting apparatus. Thus it can reduce the space and save the cost.

Position sensors 200 are provided on the preset positions of the transmitting apparatus 400 within each cabin. Each position sensor 200 is electrically connected with the industrial control computer 800, so as to ensure the accurate operation positions of corresponding items within each cabin during the conveying process of the transmitting apparatus 400. Preferably, each position sensor 200 is an infrared sensor or a radio sensor.

The cabin separating mechanism 140 is a separating valve.

In a preferred embodiment, the transmitting apparatus 400 is a belt conveyor or a roller transmitting apparatus. The belt or roller could be driven by rotating magnetic fluid within each cabin actuated by a motor outside each cabin, or directly driven by a motor provided inside of each cabin.

In a preferred embodiment, as shown in figures 1 and 2, an air pressure regulating device 110 and a ventilation device 130 are provided outside of each cabin to adjust the cabin pressure. Each air pressure regulating device 110 and each ventilation device 130 electrically connect to said industrial control computer respectively. Each air pressure regulating device 110 contains a pressure sensor for detecting the pressure of each cabin. Each pressure sensor electrically connects to the industrial control computer. Therefore, the pressure of each cabin can be controlled by the air pressure regulating device 110 and ventilation device 130 during the transfer between cabin and cabin. Sterilization devices 120 are respectively arranged outside said sterilization cabin 102, the enrichment operating cabin 105, and the buffer cabin 106 for sterilizing corresponding cabins. Thus, each cabin could be sterilized to avoid contamination.

Preferably, in the full-automatic microorganism enrichment method of the present preferred embodiment, each time the cabin separating mechanisms 140 between the preset operating position 101, the sterilization cabin 102, the middle package removing cabin 103, the inner package removing cabin 104 and the enrichment operating cabin 105 open, the pressure of the former cabin is lower than that of the latter cabin, to guarantee no bacteria being taken into the enrichment operating cabin 105. Each time the cabin separating mechanisms 140 between the enrichment operating cabin 105, the buffer cabin 106 and the positive bacteria filling cabin 107 open, the pressure of the former cabin is higher than that of the latter cabin, to guarantee no positive bacteria being adversely transferred into the enrichment operating cabin 106.

Furthermore, in the seventh step, after closing the cabin separating mechanisms 140 on both sides of the positive bacteria filling cabin 106, a further step is included, i.e. to sterilize the buffer cabin 106. The buffer cabin 106 is mainly used to transfer the positive bacteria and prevent the positive bacteria from mutual infection. This is because if the enrichment operating cabin 105 is directly connected to the positive bacteria filling cabin 106, the positive bacteria may enter into the enrichment operating cabin 105. However, since the enrichment operating cabin 105 operates continuously, keeping sterilizing it will be a waste of time. Therefore, the buffer cabin 106 could connect to the enrichment operating cabin 105 after being sterilized and the operation of the enrichment operating cabin 105 can not be interrupted. When the buffer cabin 106 is isolated with the enrichment operating cabin 105, it can be communicated with the positive bacteria filling cabin 107. After the enriched and enclosed filters 1100 are cut and transferred into the positive bacteria filling cabin 106, the buffer cabin 106 is capable of being isolated and sterilized, then it can be communicated with the enrichment operating cabin 105 to do the next transfer of the filters 1100. Therefore, it is capable of avoiding the possibility of the positive bacteria in the positive bacteria filling cabin 107 entering into the enrichment operating cabin 107, and the operation of the enrichment operating cabin 105 could not be interrupted, so that the whole system has good working continuity and high efficiency.

As shown in figures 1 and 2, in a preferred embodiment of the present invention, a middle package removing device 310 and an inner package removing device 320, which are used for removing the middle package and inner package of the filter and the filter head, are respectively arranged above said transmitting apparatus 400 within said middle package removing cabin 103 and said inner package removing cabin 104. The middle package removing device 310 and inner package removing device 320 are electrically connected with the industrial control computer respectively.

In a preferred embodiment of the present invention, as shown in figures 1 and 2, the pipeline filters 1100 are three parallel tanks with outlets on both ends, preferably the tanks are transparent. Each tank has filter film on the bottom. After being filled with the to-be-detected samples, microorganisms are enriched by the filter film. The pipelines of filter 1100 are three parallel pipelines, one end of which is provided on the top of said filter 1100 and communicated with each tank, and the other end of which is a filter needle. Said filter 1100 has a set position in the enrichment operating cabin 105, and the set position is where the bottom of each tank locates in the supporting hole of the waste liquid trough.

An enrichment operation manipulator 510 is arranged above said transmitting apparatus 400 in the enrichment operating cabin 105. The enrichment operation manipulator 510 inserts the filter needle into the to-be-detected samples and replaces the filter needle with a culture needle, then inserting it into the culture of the culture medium. The enrichment operation manipulator 510 can make the filter 1100 shift between the set position and the transmitting apparatus. An enrichment driving mechanism 500 is provided on one side of the enrichment operation manipulator 510 in the proximity of said buffer cabin for driving the to-be-detected items or culture to be filled into three parallel pipelines and enter into said filter 1100.

A packaging mechanism 530 is provided near said set position in the enrichment operating cabin 105 for packaging the bottom of each tank by the filter head after the process of filtering and enriching.

The enrichment operation manipulator 510, the enrichment driving mechanism 500 and the packaging mechanism 530 are electrically connected to the industrial control computer 800 respectively and controlled by it.

In a preferred embodiment of the present invention, said enrichment driving mechanism 500 is a peristaltic pump. A first bracket and a second bracket are provided on the pump head of the peristaltic pump for clamping the three parallel pipelines when the enrichment operation manipulator holds the filter needle to insert into the to-be-detected samples. Moreover, the first bracket is retractably fixed on one side of the peristaltic pump head near the enrichment operation manipulator 510, and the second bracket is provided on the pump head of the peristaltic pump retractably and rotatablely. The second bracket has a first position and a second position. On the first position, the second bracket is arranged side by side at the side of the first bracket for clamping the three parallel pipelines together with the first bracket. On the second position, the second bracket rotates to a location where the three parallel pipelines can be correspondingly placed into the head of the peristaltic pump. When operating, the first bracket and the second bracket clamp the three parallel pipelines respectively, then the second bracket is rotated to the second position and the first and second brackets retract back, thereby putting the three parallel pipelines into the pump head of the peristaltic pump.

Said enrichment operation manipulator 510 further includes a culture medium heat-seal mechanism for sealing at most two pipelines of the three parallel pipelines before the culture medium entering into said filter after that said filter finished filtering and enriching the to-be-detected samples. Thus, the culture medium could be selectively injected into the microorganism enriched filters. Optionally, the three parallel pipelines could be heat-sealed one, or two or none of them.

A triple pressure sensor 900 is provided in the enrichment operating cabin 105 to detect the inner pressure of said filter 1100, in order to modulate the pressure of the enrichment operating cabin by detecting the pressure in the filter.

Said peristaltic pump, the first bracket, the second bracket, the culture medium heat-seal mechanism and the triplet pressure sensor 900 are electrically connected with the industrial control computer 800, respectively.

Therefore, the operation in said enrichment operating cabin 105 comprises following steps:
Said filter 1100 is placed on the preset position by the enrichment operation manipulator 510, and then the filter needle is inserted into the to-be-detected samples. The three parallel pipelines are placed into the pump head of the peristaltic pump by the first and second brackets. The peristaltic pump starts to work, the filter 1100 filters the fluid of the to-be-detected samples 1000 and the possible microorganisms are enriched on the filter film. Then the enrichment operation is completed. After that, the packaging mechanism 530 encloses the bottom of each tank by the filter head to complete the packaging operation. Moreover, one pipeline is selected to be closed by the culture medium heat-seal mechanism. The enrichment operation manipulator 510 replaces the filter needle with a culture needle and inserts it into the culture of the culture medium. The culture is injected into the two pipelines by the peristaltic pump and enriched in two corresponding tanks. Above all, enrichment and packaging of the filter and injection of the culture are completed in the enrichment operating cabin 105.

The industry control computer performs internal control programs according to the signals detected by each sensor to respectively control the pressure in each cabin, the transmission of the transmitting apparatus and corresponding actions of actuators in each cabin accordingly. The control programs belong to the common knowledge known by the skilled in the art, which are not created by the present inventor and therefore will not be described in detail here.

Accordingly, in one preferred embodiment of the full-automatic microorganism enrichment method of the present invention, said filters are three parallel tanks, in two of which the culture could be filled in.

Preferably, in said fifth step, used samples and culture medium are transferred back to the inner package removing cabin by the transmitting apparatus 400 for recycling.

Preferably, as shown by figures 1 and 2, in one preferred embodiment of the present invention, a pipeline cutting mechanism 520 are provided above the transmitting apparatus 400 in said buffer cabin 106, in order to package the pipeline of the filter.

The pipeline cutting mechanism 520 is electrically connected with the industrial control computer 800.

Preferably, as depicted in figures 1 and 2, in one preferred embodiment of the present invention, said positive bacteria filling cabin 107 has a heating function, so that the positive bacteria can be cultured in the positive bacteria filling cabin 107 immediately after the completion of the positive bacteria filling. A positive bacteria filling mechanism 700 is provided above the transmitting apparatus 400 in the positive bacteria filling cabin 107. A temperature control apparatus is further provided in the positive bacteria filling cabin.

The positive bacteria filling mechanism 700 and the temperature control apparatus are electrically connected with the industrial control computer 800, respectively.

Preferably, the industrial control computer 800 could be a form of the host computer. Each cabin may have separate control unit.

In summary, the present invention provides a full-automatic microorganism detecting enrichment system and the enrichment method, which can achieve a fully automated enrichment of the microorganism in the to-be-detected samples, needs no manual operation, and saves time and labor force. Further, it can effectively avoid false positive or false negative result caused by an effect of human factors, and achieve sterile and automatic operation with an accurate detecting result. The enrichment process is less error-prone and is able to be operated continuously, with no need to pause and sterile the enrichment operating cabin after stopping the enrichment operation, consequently it has a better implementation effect.

## Claims

1. A full-automatic microorganism detecting enrichment system, **characterized in that** the system comprises:
a preset operating position (101), for placing to-be-detected samples, a culture medium containing culture, a pipeline filter specially for collecting bacteria and a filter head; the filter utilizing a filter film for filtering the to-be-detected samples and enriching the microorganisms possibly contained in the to-be-detected samples;
a sterilization cabin (102) for conducting sterilization, a middle package removing cabin (103) for removing the middle package, an inner package removing cabin (104) for removing the inner package, an enrichment operating cabin (105) for injecting the to-be-detected samples into a filter to filter the enriched microorganism, enclosing the filter bottom by a filter head, and selectively injecting the culture into the filter, a buffer cabin (106) for cutting the filter pipeline after the enrichment, and a positive bacteria filling cabin for filling the positive bacteria, being disposed adjacent successively behind the preset operating position; above said cabins being connected and separated respectively by a cabin separating mechanism;
a transmitting apparatus (400), for transmitting the to-be-detected samples placed on the preset operating position, the culture medium, the filter and the filter head into each corresponding cabin respectively, the transmitting apparatus starting from the preset operating position and running through above said cabins; and
a industrial control computer (800), for electrically connecting above each cabin, each cabin separating mechanism and the transmitting apparatus.

2. The full-automatic microorganism detecting enrichment system according to claim 1, wherein a fixing device is provided on the transmitting apparatus for fixing the to-be-detected samples, the culture medium containing the culture, the filter and the filter head, and/or
the transmitting apparatus could be divided into a to-be-detected sample transmitting apparatus, a culture medium transmitting apparatus, a filter transmitting apparatus and a filter head transmitting apparatus; and/or
position sensors are provided on the preset positions of the transmitting apparatus within each cabin and electrically connected with the industrial control computer; and/or
the transmitting apparatus is a belt conveyor or a roller transmitting apparatus; the belt or roller could be driven by rotating magnetic fluid within each cabin actuated by a motor outside each cabin, or directly driven by a motor provided inside of each cabin; and/or
each cabin separating mechanism is a separating valve.

3. The full-automatic microorganism detecting enrichment system according to claim 1, wherein an air pressure regulating device (110) and a ventilation device (130) are provided outside of each cabin to adjust the cabin pressure; each air pressure regulating device and each ventilation device electrically connect to said industrial control computer respectively; each air pressure regulating device contains a pressure sensor for detecting the pressure of each cabin, and each pressure sensor electrically connects to the industrial control computer; and/or
sterilization devices (120) are respectively arranged outside said sterilization cabin, the enrichment operating cabin, and the buffer cabin for sterilizing corresponding cabins.

4. The full-automatic microorganism detecting enrichment system according to claim 1, wherein a middle package removing device (310) and an inner package removing device (320), which are used for removing the middle package and inner package of the filter and the filter head, are respectively arranged above said transmitting apparatus within said middle package removing cabin and said inner package removing cabin; the inner package removing device and the middle package removing device are electrically connected with the industrial control computer respectively

5. The full-automatic microorganism detecting enrichment system according to claim 1, wherein the pipeline filters are three parallel tanks with outlets on both ends, each tank has filter film on the bottom, after being filled with the to-be-detected samples, microorganisms are enriched by the filter film; the pipelines of filter are three parallel pipelines, one end of which is provided on the top of said filter and communicated with each tank, and the other end of which is a filter needle; said filter has a set position in the enrichment operating cabin, and the set position is where the bottom of each tank locates in the supporting hole of the waste liquid trough;
an enrichment operation manipulator (510) is arranged above said transmitting apparatus in the enrichment operating cabin, the enrichment operation manipulator inserts the filter needle into the to-be-detected samples and replaces the filter needle with a culture needle, then inserting it into the culture of the culture medium; the enrichment operation manipulator can make the filter shift between the set position and the transmitting apparatus; an enrichment driving mechanism (500) is provided on one side of the enrichment operation manipulator in the proximity of said buffer cabin for driving the to-be-detected items or culture to be filled into three parallel pipelines and to enter into said filter;
a packaging mechanism (530) is provided near said set position in the enrichment operating cabin for packaging the bottom of each tank by the filter head after the process of filtering and enriching;
the enrichment operation manipulator, the enrichment driving mechanism and the packaging mechanism are electrically connected to the industrial control computer respectively.

6. The full-automatic microorganism detecting enrichment system according to claim 5, wherein said enrichment driving mechanism (500) is a peristaltic pump, a first bracket and a second bracket are provided on the pump head of the peristaltic pump for clamping the three parallel pipelines when the enrichment operation manipulator holds the filter needle to insert into the to-be-detected samples, the first bracket is retractably fixed on one side of the peristaltic pump head near the enrichment operation manipulator, the second bracket is provided on the pump head of the peristaltic pump retractably and rotatablely; the second bracket has a first position and a second position, on the first position, the second bracket is arranged side by side at the side of the first bracket for clamping the three parallel pipelines together with the first bracket, on the second position, the second bracket rotates to a location where the three parallel pipelines can be correspondingly placed into the pump head of the peristaltic pump; when operating, the first bracket and the second bracket clamp the three parallel pipelines respectively, then the second bracket is rotated to the second position and the first and second brackets retract back, thereby putting the three parallel pipelines into the pump head of the peristaltic pump
said enrichment operation manipulator (510) further includes a culture medium heat-seal mechanism for sealing at most two pipelines of the three parallel pipelines before the culture medium entering into said filter after that said filter finished filtering and enriching the to-be-detected samples, thereby the culture medium could be selectively injected into the microorganism enriched filters;
a triple pressure sensor (900) is provided in the enrichment operating cabin to detect the inner pressure of said filter;
the peristaltic pump, the first bracket, the second bracket, the culture medium heat-seal mechanism and the triplet pressure sensor are electrically connected with the industrial control computer, respectively.

7. The full-automatic microorganism detecting enrichment system according to claim 1, wherein a pipeline cutting mechanism (5520) is provided above the transmitting apparatus in said buffer cabin, and
the pipeline cutting mechanism is electrically connected with the industrial control computer.

8. The full-automatic microorganism detecting enrichment system according to claim 1, wherein said positive bacteria filling cabin has a heating function, a positive bacteria filling mechanism (700) is provided above the transmitting apparatus in the positive bacteria filling cabin; a temperature control apparatus is further provided in the positive bacteria filling cabin;
the positive bacteria filling mechanism and the temperature control apparatus are electrically connected with the industrial control computer, respectively; and/or
the industrial control computer is a form of the host computer, and each cabin has separate control unit.

9. A full-automatic microorganism enriching method, **characterized in that** it comprises the following steps of:
the first step: placing to-be-detected samples, the culture medium containing culture, the pipeline filter specially for collecting bacteria and the filter head on the transmitting apparatus of the preset operating position ;
the second step: opening the cabin separating mechanism between the preset operating position and the sterilization cabin, transmitting the to-be-detected samples, the culture medium containing culture, the pipeline filter specially for collecting bacteria and the filter head into said sterilization cabin, closing the cabin separating mechanisms on both sides of the sterilization cabin and carrying out sterilization, then ventilating for balancing the pressure in the sterilization cabin after finishing the sterilization;
the third step: opening the cabin separating mechanism between the sterilization cabin and the middle package removing cabin, transmitting the to-be-detected samples, the culture medium containing culture, the pipeline filter specially for collecting bacteria and the filter head into the middle package removing cabin, closing the cabin separating mechanisms on both sides of the middle package removing cabin, and removing the middle packages of the filter and the filter head;
the fourth step: opening the cabin separating mechanism between the middle package removing cabin and the inner package removing cabin, transmitting the to-be-detected samples, the culture medium containing culture, the pipeline filter specially for collecting bacteria and the filter head into the inner package removing cabin, closing the cabin separating mechanisms on both sides of inner package removing cabin, and removing the inner package of the filter and the filter head;
the fifth step: opening the cabin separating mechanism between the inner package removing cabin and the enrichment operating cabin, transmitting the to-be-detected samples, the culture medium containing culture, the pipeline filter specially for collecting bacteria and the filter head into the enrichment operating cabin, closing the cabin separating mechanisms on both sides of the enrichment operating cabin, filtering and enriching the microorganisms contained in the to-be-detected samples in the filter, enclosing the filter bottom by the filter head, then selectively injecting the culture into the filter, placing the enriched and enclosed filter on the transmitting apparatus, obtaining appropriate number of the enriched and enclosed filters;
the sixth step: opening the cabin separating mechanism between the enrichment operating cabin and the buffer cabin, transmitting the enriched and enclosed filters into the buffer cabin, closing the cabin separating mechanisms on both sides of buffer cabin, cutting the pipeline of enriched and enclosed filters; and
the seventh step, opening the cabin separating mechanism between the buffer cabin and the positive bacteria filling cabin, transmitting the enriched and enclosed filters into the positive bacteria filling cabin, closing the cabin separating mechanisms on both sides of the positive bacteria filling cabin, filling the positive bacteria into the enriched and enclosed filters after finishing the pipeline cutting, then completing the microorganism enrichment of the detected samples.

10. The full-automatic microorganism enriching method according to claim 9, wherein each time the cabin separating mechanism between the preset operating position, the sterilization cabin, the middle package removing cabin, the inner package removing cabin and the enrichment operating cabin open, the pressure of the former cabin is lower than that of the latter cabin, to guarantee no bacteria being taken into the enrichment operating cabin; and each time the cabin separating mechanisms between the enrichment operating cabin, the buffer cabin and the positive bacteria filling cabin open, the pressure of the former cabin is higher than that of the latter cabin, to guarantee no positive bacteria being adversely transferred into the enrichment operating cabin; and/or
in said seventh step, after closing the cabin separating mechanisms on both sides of the positive bacteria filling cabin, a further step is included: sterilizing the buffer cabin; and/or
said filters are three parallel tanks with outlets on both ends, the culture are selectively injected into at most two of those tanks; and/or
in said fifth step, used samples and culture medium are transferred back to the inner package removing cabin by the transmitting apparatus for recycling.

## Patentansprüche

1. Vollautomatisches Detektions- und Anreicherungssystem für Mikroorganismen, **dadurch gekennzeichnet, dass** das System umfasst:
eine vorgegebene Betriebsposition (101) zum Platzieren von zu detektierenden Proben, ein Kulturmedium, das die Kultur enthält, einen Leitungsfilter, der speziell zum Sammeln von Bakterien geeignet ist, und einen Filterkopf, wobei der Filter eine Filterfolie zum Filtern der zu detektierenden Proben und zum Anreichern der in den zu detektierenden Proben möglicherweise enthaltenen Mikroorganismen verwendet wird,
eine Sterilisationskammer (102) zum Durchführen der Sterilisation, eine mittlere Verpackungs-Entfernungskammer (103) zum Entfernen der mittleren Verpackung, eine innere Verpackungs-Entfernungskammer (104) zum Entfernen der inneren Verpackung, eine Anreicherungsdurchführungskammer (105) zum Einspritzen der zu detektierenden Proben in einen Filter zum Filtern des angereicherten Mikroorganismus, zum Umschließen des Filterbodens durch einen Filterkopf und zum selektiven Einspritzen der Kultur in den Filter, eine Pufferkammer (106) zum Durchschneiden der Filterleitung nach der Anreicherung und eine positive Bakterien-Abfüllkammer zum Abfüllen der positiven Bakterien, die nacheinander hinter der vorgegebenen Betriebsposition angeordnet sind, wobei die oben genannten Kammern durch einen Kammertrennmechanismus verbunden bzw. getrennt sind,
eine Weiterleitungsvorrichtung (400) zum Weiterleiten der zu detektierenden Proben, die auf der vorgegebenen Betriebsposition platziert sind, dem Kulturmedium, dem Filter und dem Filterkopf in die jeweilige entsprechende Kammer, wobei die Weiterleitungsvorrichtung von der vorgegebenen Betriebsposition ausgeht und durch die oben genannten Kammern läuft, und
einen industriellen Steuerungscomputer (800) zum elektrischen Verbinden mit jeder oben genannten Kammer, jedem Kammertrennmechanismus und der Weiterleitungsvorrichtung.

2. Vollautomatisches Detektions- und Anreicherungssystem für Mikroorganismen nach Anspruch 1, wobei an der Weiterleitungsvorrichtung eine Fixiervorrichtung zum Fixieren der zu detektierenden Proben, des Kulturmediums, das die Kultur enthält, des Filters und des Filterkopfes vorgesehen ist, und/oder
wobei die Weiterleitungsvorrichtung in eine Weiterleitungsvorrichtung für zu detektierende Proben, eine Kulturmedium-Weiterleitungsvorrichtung, eine Filter-Weiterleitungsvorrichtung und eine Filterkopf-Weiterleitungsvorrichtung unterteilt werden kann, und/oder
wobei Positionssensoren an den vorgegebenen Positionen der Weiterleitungsvorrichtung innerhalb jeder Kammer vorgesehen und elektrisch mit dem industriellen Steuerungscomputer verbunden sind, und/oder
wobei die Weiterleitungsvorrichtung ein Transportband oder eine Walzenweiterleitungsvorrichtung ist, wobei das Transportband oder die Walze durch umlaufendes magnetisches Fluid innerhalb jeder Kammer angetrieben werden können, das durch einen Motor außerhalb jeder Kammer in Bewegung gesetzt wird, oder direkt durch einen Motor angetrieben werden, der innerhalb jeder Kammer vorgesehen ist, und/oder
wobei jeder Kammer-Trennmechanismus ein Trennschieber ist.

3. Vollautomatisches Detektions- und Anreicherungssystem für Mikroorganismen nach Anspruch 1, wobei eine Luftdruck-Regelungsvorrichtung (110) und eine Belüftungsvorrichtung (130) außerhalb jeder Kammer vorgesehen sind, um den Kammerdruck einzustellen, wobei jede Luftdruck-Regelungsvorrichtung und jede Belüftungsvorrichtung jeweils elektrisch mit dem industriellen Steuerungscomputer verbunden sind, wobei jede Luftdruck-Regelungsvorrichtung einen Drucksensor zum Erfassen des Drucks in jeder Kammer enthält, und jeder Drucksensor elektrisch mit dem industriellen Steuerungscomputer verbunden ist, und/oder
Sterilisationsvorrichtungen (120) jeweils außerhalb der Sterilisationskammer, der Anreicherungsdurchführungskammer und der Pufferkammer zum Sterilisieren der entsprechenden Kammern angeordnet sind.

4. Vollautomatisches Detektions- und Anreicherungssystem für Mikroorganismen nach Anspruch 1, wobei eine mittlere Verpackungs-Entfernungsvorrichtung (310) und eine innere Verpackungs-Entfernungsvorrichtung (320), die zum Entfernen der mittleren Verpackung und der inneren Verpackung des Filters und des Filterkopfes verwendet werden, jeweils oberhalb der Weiterleitungsvorrichtung innerhalb der mittleren Verpackungs-Entfernungskammer und der inneren Verpackungs-Entfernungskammer angeordnet sind, wobei die innere Verpackungs-Entfernungsvorrichtung und die mittlere Verpackungs-Entfernungsvorrichtung jeweils elektrisch mit dem industriellen Steuerungscomputer verbunden sind.

5. Vollautomatisches Detektions- und Anreicherungssystem für Mikroorganismen nach Anspruch 1, wobei die Leitungsfilter drei parallele Tanks mit Auslässen an beiden Enden sind, jeder Tank eine Filterfolie auf dem Boden aufweist, wobei nachdem er mit den zu detektierenden Proben gefüllt wurde, Mikroorganismen durch die Filterfolie angereichert werden, die Leitungen des Filters drei parallele Leitungen sind, von denen ein Ende auf der Oberseite des Filters vorgesehen und mit jedem Tank verbunden ist und das andere Ende davon eine Filternadel ist, wobei der Filter eine Sollposition in der Anreicherungsdurchführungskammer aufweist und die Sollposition der Ort ist, an dem sich der Boden jedes Tanks in der Halteöffnung der Abwasserwanne befindet,
wobei ein Anreicherungsdurchführungsmanipulator (510) oberhalb der Weiterleitungsvorrichtung in der Anreicherungsdurchführungskammer angeordnet ist, wobei der Anreicherungsdurchführungsmanipulator die Filternadel in die zu detektierenden Proben einsetzt und die Filternadel durch eine Kulturnadel ersetzt, sie dann in die Kultur des Kulturmediums einsetzt, wobei der Anreicherungsdurchführungsmanipulator den Filter zwischen der Sollposition und der Weiterleitungsvorrichtung verschieben kann, wobei ein Anreicherungsantriebsmechanismus (500) auf einer Seite des Anreicherungsdurchführungsmanipulators in der Nähe der Pufferkammer vorgesehen ist, um die zu detektierenden Produkte oder Kulturen anzutreiben, die in drei parallele Leitungen gefüllt werden und in den Filter eintreten sollen,
wobei ein Verpackungsmechanismus (530) in der Nähe der Sollposition in der Anreicherungsdurchführungskammer vorgesehen ist, um den Boden jedes Tanks durch den Filterkopf nach dem Filtern und Anreichern zu verpacken,
wobei der Anreicherungsdurchführungsmanipulator, der Anreicherungsantriebsmechanismus und der Verpackungsmechanismus jeweils elektrisch mit dem industriellen Steuerungscomputer verbunden sind.

6. Vollautomatisches Detektions- und Anreicherungssystem für Mikroorganismen nach Anspruch 5, wobei der Anreicherungsantriebsmechanismus (500) eine peristaltische Pumpe ist, eine erste Halterung und eine zweite Halterung an dem Pumpenkopf der peristaltischen Pumpe zum Festklemmen der drei parallelen Leitungen vorgesehen sind, wenn der Anreicherungsdurchführungsmanipulator die Filternadel hält, um sie in die zu detektierenden Proben einzusetzen, die erste Halterung auf einer Seite des peristaltischen Pumpenkopfes in der Nähe des Anreicherungsoperationsmanipulators zurückziehbar befestigt ist, die zweite Halterung auf dem Pumpenkopf der peristaltischen Pumpe zurückziehbar und drehbar vorgesehen ist, die zweite Halterung eine erste Position und eine zweite Position aufweist, wobei in der ersten Position die zweite Halterung nebeneinanderliegend an der Seite der ersten Halterung zum Festklemmen der drei parallelen Leitungen zusammen mit der ersten Halterung angeordnet ist, in der zweiten Position die zweite Halterung zu einer Position dreht, an der die drei parallelen Leitungen entsprechend in den Pumpenkopf der peristaltischen Pumpe eingesetzt werden können, wobei im Betrieb die erste Halterung und die zweite Halterung die drei parallelen Leitungen festklemmen, danach wird die zweite Halterung in die zweite Position gedreht und die erste und zweite Halterung ziehen sich zurück, wodurch die drei parallelen Leitungen in den Pumpenkopf der peristaltischen Pumpe eingelegt werden,
wobei der Anreicherungsdurchführungsmanipulator (510) ferner einen Kulturmedium-Heißversiegelungsmechanismus zum Verschließen von höchstens zwei Leitungen der drei parallelen Leitungen vor dem Eintreten des Kulturmediums in den Filter umfasst, nachdem der Filter das Filtern beendet und die zu detektierenden Proben angereichert hat, wodurch das Kulturmedium selektiv in die mit Mikroorganismen angereicherten Filter injiziert werden kann,
ein Dreifachdrucksensor (900) in der Anreicherungsdurchführungskammer vorgesehen ist, um den Innendruck des Filters zu erfassen,
wobei die peristaltische Pumpe, die erste Halterung, die zweite Halterung, der Kulturmedium-Heißversiegelungsmechanismus und der Dreifachdrucksensor elektrisch mit dem industriellen Steuerungscomputer verbunden sind.

7. Vollautomatisches Detektions- und Anreicherungssystem für Mikroorganismen nach Anspruch 1, wobei ein Leitungsschneidemechanismus (5520) oberhalb der Weiterleitungsvorrichtung in der Pufferkammer vorgesehen ist, und
der Leitungsschneidemechanismus elektrisch mit dem industriellen Steuerungscomputer verbunden ist.

8. Vollautomatisches Detektions- und Anreicherungssystem für Mikroorganismen nach Anspruch 1, wobei die positive Bakterien-Abfüllkammer eine Heizfunktion aufweist, ein positiver Bakterien-Abfüllmechanismus (700) oberhalb der Weiterleitungsvorrichtung in der positiven Bakterien-Abfüllkammer vorgesehen ist, eine Temperaturkontrollvorrichtung ferner in der positiven Bakterienfüllkammer vorgesehen ist,
wobei der positive Bakterien-Abfüllmechanismus und die Temperaturkontrollvorrichtung elektrisch mit dem industriellen Steuerungscomputer verbunden sind, und/oder
der industrielle Steuerungscomputer eine Form des Hauptrechners ist und jede Kammer eine separate Steuereinheit aufweist.

9. Vollautomatisches Verfahren zur Anreicherung von Mikroorganismen, **dadurch gekennzeichnet, dass** es die folgenden Schritte umfasst:
den ersten Schritt: Platzieren der zu detektierenden Proben, des Kulturmediums, das die Kultur enthält, des Leitungsfilters speziell zum Sammeln von Bakterien und den Filterkopf auf die Weiterleitungsvorrichtung der vorgegebenen Betriebsposition,
den zweiten Schritt: Öffnen des Kammertrennmechanismus zwischen der vorgegebenen Betriebsposition und der Sterilisationskammer, Weiterleiten der zu detektierenden Proben, des Kulturmediums, das die Kultur enthält, des Leitungsfilters speziell zum Sammeln von Bakterien und des Filterkopfes in die Sterilisationskammer, Schließen der Kammertrennmechanismen auf beiden Seiten der Sterilisationskammer und Durchführen der Sterilisation, danach Belüften zum Ausgleich des Drucks in der Sterilisationskammer nach Abschluss der Sterilisation,
den dritten Schritt: Öffnen des Kammertrennmechanismus zwischen der Sterilisationskammer und der mittleren Verpackungs-Entfernungskammer, Weiterleiten der zu detektierenden Proben, des Kulturmediums, des Leitungsfilters speziell zum Sammeln von Bakterien und des Filterkopfes in die mittlere Verpackungs-Entfernungskammer, Schließen der Kammertrennmechanismen auf beiden Seiten der mittleren Verpackungs-Entfernungskammer und Entfernen der mittleren Verpackungen des Filters und des Filterkopfes,
den vierten Schritt: Öffnen des Kammertrennmechanismus zwischen der mittleren Verpackungs-Entfernungskammer und der inneren Verpackungs-Entfernungskammer, Weiterleiten der zu detektierenden Proben, des Kulturmediums, des Leitungsfilters speziell zum Sammeln von Bakterien und des Filterkopfes in die innere Verpackungs-Entfernungskammer, Schließen der Kammertrennmechanismen auf beiden Seiten der inneren Verpackungs-Entfernungskammer und Entfernen der inneren Verpackung des Filters und des Filterkopfes,
den fünften Schritt: Öffnen des Kammertrennmechanismus zwischen der inneren Verpackungs-Entfernungskammer und der Anreicherungsdurchführungskammer, Weiterleiten der zu detektierenden Proben, des Kulturmediums, das die Kultur enthält, des Leitungsfilters speziell zum Sammeln von Bakterien und des Filterkopfes in die Anreicherungsdurchführungskammer, Schließen der Kammertrennmechanismen auf beiden Seiten der Anreicherungsdurchführungskammer, Filtern und Anreichern der Mikroorganismen, die in den zu detektierenden Proben enthalten sind, in dem Filter, Umschließen des Filterbodens durch den Filterkopf, danach selektives Einspritzen der Kultur in den Filter, Platzieren des angereicherten und umschlossenen Filters auf der Weiterleitungsvorrichtung, Erhalten einer entsprechenden Anzahl der angereicherten und umschlossenen Filter,
den sechsten Schritt: Öffnen des Kammertrennmechanismus zwischen der Anreicherungsdurchführungskammer und der Pufferkammer, Weiterleiten der angereicherten und umschlossenen Filter in die Pufferkammer, Schließen der Kammertrennmechanismen auf beiden Seiten der Pufferkammer, Durchschneiden der Leitung der angereicherten und umschlossenen Filter, und
den siebten Schritt, Öffnen des Kammertrennmechanismus zwischen der Pufferkammer und der positive Bakterien-Abfüllkammer, Weiterleiten der angereicherten und umschlossenen Filter in die positive Bakterien-Abfüllkammer, Schließen der Kammertrennmechanismen auf beiden Seiten der positive Bakterien-Abfüllkammer, Abfüllen der positiven Bakterien in die angereicherten und umschlossenen Filter nach Beendigung des Leitungsschneidens, danach Abschließen der Mikroorganismenanreicherung der detektierten Proben.

10. Vollautomatisches Anreicherungsverfahren für Mikroorganismen nach Anspruch 9, wobei jedes Mal, wenn der Kammertrennmechanismus zwischen der vorgegebenen Betriebsposition, der Sterilisationskammer, der mittleren Verpackungs-Entfernungskammer, der inneren Verpackungs-Entfernungskammer und der Anreicherungsdurchführungskammer sich öffnet, der Druck der ersten Kammer niedriger ist als derjenige der zweiten Kammer, um zu gewährleisten, dass keine Bakterien in die Anreicherungsdurchführungskammer gelangen, und jedes Mal, wenn sich die Kammertrennmechanismen zwischen der Anreicherungsdurchführungskammer, der Pufferkammer und der positive Bakterien-Abfüllkammer öffnen, der Druck der ersten Kammer höher ist als derjenige der zweiten Kammer, um zu gewährleisten, dass keine positiven Bakterien nachteilig in die Anreicherungsdurchführungskammer übertragen werden, und/oder
in dem siebten Schritt nach dem Schließen der Kammertrennmechanismen auf beiden Seiten der positive Bakterien-Abfüllkammer ein weiterer Schritt eingeschlossen ist: Sterilisieren der Pufferkammer, und/oder
die Filter drei parallele Behälter mit Auslässen an beiden Enden sind, wobei die Kultur selektiv in höchstens zwei dieser Behälter eingespritzt wird, und/oder
in dem fünften Schritt verwendete Proben und Kulturmedium durch die Weiterleitungsvorrichtung zum Recycling zurück in die innere Verpackungs-Entfernungskammer transportiert werden.

## Revendications

1. Système entièrement automatique d'enrichissement par détection de microorganisme, **caractérisé en ce que** le système comprend :
une position de fonctionnement préréglée (101) pour placer des échantillons à détecter, un milieu de culture contenant une culture, un filtre de canalisation spécialement pour collecter des bactéries et une tête de filtre ; le filtre utilisant un film filtrant pour filtrer les échantillons à détecter et enrichir les microorganismes éventuellement contenus dans les échantillons à détecter ;
une cabine de stérilisation (102) pour réaliser la stérilisation, une cabine de retrait d'emballage central (103) pour retirer l'emballage central, une cabine de retrait d'emballage interne (104) pour retirer l'emballage interne, une cabine de commande d'enrichissement (105) pour injecter les échantillons à détecter dans un filtre afin de filtrer le microorganisme enrichi, entourer le fond de filtre par une tête de filtre, et sélectivement injecter la culture dans le filtre, une cabine tampon (106) pour coupler la canalisation de filtre après l'enrichissement, et une cabine de remplissage de bactéries positives pour verser les bactéries positives, étant disposée de manière adjacente successivement derrière la position de fonctionnement préréglée ; au-dessus lesdites cabines sont raccordées et séparées respectivement par un mécanisme de séparation de cabine ;
un appareil de transmission (400), pour transmettre les échantillons à détecter placés dans la position de fonctionnement préréglée, le milieu de culture, le filtre et la tête de filtre dans chaque cabine correspondante, respectivement, l'appareil de transmission commençant à partir de la position de fonctionnement préréglée et s'étendant au-dessus desdites cabines ; et
un ordinateur de commande industriel (800) pour raccorder électriquement au-dessus de chaque cabine, chaque mécanisme de séparation de cabine et l'appareil de transmission.

2. Système entièrement automatique de détection et d'enrichissement de microorganisme selon la revendication 1, dans lequel un dispositif de fixation est prévu sur l'appareil de transmission pour fixer les échantillons à détecter, le milieu de culture contenant la culture, le filtre et la tête de filtre, et/ou
l'appareil de transmission peut être divisé en un appareil de transmission d'échantillon à détecter, un appareil de transmission de milieu de culture, un appareil de transmission de filtre et un appareil de transmission de tête de filtre ; et/ou
des capteurs de position sont prévus dans les positions préréglées de l'appareil de transmission à l'intérieur de chaque cabine et électriquement raccordés avec l'ordinateur de commande industriel ; et/ou
l'appareil de transmission est une courroie transporteuse ou un appareil de transmission à rouleau ; la courroie ou le rouleau peut être entraîné(e) en faisant tourner le fluide magnétique à l'intérieur de chaque cabine actionnée par un moteur à l'extérieur de chaque cabine, ou directement entraîné par un moteur prévu à l'intérieur de chaque cabine ; et/ou
chaque mécanisme de séparation de cabine est une valve de séparation.

3. Système entièrement automatique d'enrichissement par détection de microorganisme selon la revendication 1, dans lequel un dispositif de régulation de pression d'air (110) et un dispositif de ventilation (130) sont prévus à l'extérieur de chaque cabine pour ajuster la pression de cabine ; chaque dispositif de régulation de pression d'air et chaque dispositif de ventilation se raccordent électriquement audit ordinateur de commande industriel respectivement ; chaque dispositif de régulation de pression d'air contient un capteur de pression pour détecter la pression de chaque cabine, et chaque capteur de pression se raccorde électriquement à l'ordinateur de commande industriel ; et/ou
des dispositifs de stérilisation (120) sont agencés respectivement à l'extérieur de ladite cabine de stérilisation, la cabine de commande d'enrichissement et la cabine tampon pour stériliser des cabines correspondantes.

4. Système entièrement automatique d'enrichissement par détection de microorganisme selon la revendication 1, dans lequel un dispositif de retrait d'emballage central (310) et un dispositif de retrait d'emballage interne (320), qui sont utilisés pour retirer l'emballage central et l'emballage interne du filtre et de la tête de filtre, sont respectivement agencés au-dessus dudit appareil de transmission à l'intérieur de ladite cabine de retrait d'emballage central et ladite cabine de retrait d'emballage interne ; le dispositif de retrait d'emballage interne et le dispositif de retrait d'emballage central sont électriquement raccordés avec l'ordinateur de commande industriel respectivement.

5. Système entièrement automatique d'enrichissement par détection de microorganisme selon la revendication 1, dans lequel les filtres de canalisation sont trois réservoirs parallèles avec des sorties aux deux extrémités, chaque réservoir a le film filtrant au fond, après avoir été rempli avec les échantillons à détecter, les microorganismes sont enrichis par le film filtrant ; les canalisations de filtre sont trois canalisations parallèles, dont une extrémité est prévue sur le dessus dudit filtre et communique avec chaque réservoir et dont l'autre extrémité est une aiguille filtrante ; ledit filtre a une position de consigne dans la cabine de commande d'enrichissement, et la position de consigne est à l'endroit où le fond de chaque réservoir est positionné dans le trou de support de la cuve de liquide résiduel ;
un manipulateur d'opération d'enrichissement (510) est agencé au-dessus dudit appareil de transmission dans la cabine de commande d'enrichissement, le manipulateur d'opération d'enrichissement insère l'aiguille de filtre dans les échantillons à détecter et remplace l'aiguille filtrante par une aiguille de culture, l'insérant ensuite dans la culture du milieu de culture ; le manipulateur d'opération d'enrichissement peut effectuer le changement de filtre entre la position de consigne et l'appareil de transmission ; un mécanisme d'entraînement d'enrichissement (500) est prévu sur un côté du manipulateur d'opération d'enrichissement à proximité de ladite cabine tampon pour entraîner les produits ou culture à détecter pour être versés dans trois canalisations parallèles et pour pénétrer dans ledit filtre ;
un mécanisme d'emballage (530) est prévu à proximité de ladite position de consigne dans la cabine de commande d'enrichissement pour emballer le fond de chaque réservoir par la tête de filtre après le processus de filtration et d'enrichissement ;
le manipulateur d'opération d'enrichissement, le mécanisme d'entraînement d'enrichissement et le mécanisme d'emballage sont respectivement électriquement raccordés à l'ordinateur de commande industriel.

6. Système entièrement automatique d'enrichissement par détection de microorganisme selon la revendication 5, dans lequel ledit mécanisme d'entraînement d'enrichissement (500) est une pompe péristaltique, une première console et une seconde console sont prévues sur la tête de pompe de la pompe péristaltique pour serrer les trois canalisations parallèles lorsque le manipulateur d'opération d'enrichissement maintient l'aiguille filtrante pour l'insertion dans les échantillons à détecter, la première console est fixée par rétraction sur un côté de la tête de pompe péristaltique à proximité du manipulateur d'opération d'enrichissement, la seconde console est prévue sur la tête de pompe de la pompe péristaltique de manière rétractable et rotative ; la seconde console a une première position et une seconde position, sur la première position, la seconde console est agencée côte à côte sur le côté de la première console pour serrer les trois canalisations parallèles ensemble avec la première console, sur la seconde position, la seconde console tourne à un emplacement où les trois canalisations parallèles peuvent être placées, de manière correspondante, dans la tête de pompe de la pompe péristaltique ; lors du fonctionnement, la première console et la seconde console serrent les trois canalisations parallèles respectivement, ensuite la seconde console est entraînée en rotation dans la seconde position et les première et seconde consoles se rétractent, plaçant ainsi les trois canalisations parallèles dans la tête de pompe de la pompe péristaltique,
ledit manipulateur d'opération d'enrichissement (510) comprend en outre un mécanisme de thermosoudage de milieu de culture pour sceller au maximum deux canalisations des trois canalisations parallèles avant que le milieu de culture n'entre dans ledit filtre après que le filtre a fini la filtration et l'enrichissement des échantillons à détecter, ainsi le milieu de culture peut être sélectivement injecté dans les filtres enrichis en microorganismes ;
un triple capteur de pression (900) est prévu dans la cabine d'opération d'enrichissement pour détecter la pression interne dudit filtre ;
la pompe péristaltique, la première console, la seconde console, le mécanisme de thermosoudage de milieu de culture et le triple capteur de pression sont électriquement raccordés avec l'ordinateur de commande industriel, respectivement.

7. Système entièrement automatique de détection et d'enrichissement de microorganisme selon la revendication 1, dans lequel un mécanisme de coupe de canalisation (5520) est prévu au-dessus de l'appareil de transmission dans ladite cabine tampon, et
le mécanisme de coupe de canalisation est électriquement raccordé avec l'ordinateur de commande industriel.

8. Système entièrement automatique de détection et d'enrichissement de microorganisme selon la revendication 1, dans lequel ladite cabine de remplissage de bactéries positives a une fonction de chauffage, un mécanisme de remplissage de bactéries positives (700) est prévu au-dessus de l'appareil de transmission dans la cabine de remplissage de bactéries positives ; un appareil de contrôle de température est en outre prévu dans la cabine de remplissage de bactéries positives ;
le mécanisme de remplissage de bactéries positives et l'appareil de contrôle de température sont électriquement raccordés à l'ordinateur de commande industriel respectivement ; et/ou
l'ordinateur de commande industriel se présente sous la forme d'un ordinateur hôte, et chaque cabine a une unité de commande séparée.

9. Procédé entièrement automatique d'enrichissement de microorganisme, **caractérisé en ce qu'**il comprend les étapes suivantes :
la première étape : placer les échantillons à détecter, le milieu de culture contenant la culture, le filtre de canalisation pour collecter les bactéries et la tête de filtre sur l'appareil de transmission de la position de fonctionnement préréglée ;
la deuxième étape : ouvrir le mécanisme de séparation de cabine entre la position de fonctionnement préréglée et la cabine de stérilisation, transmettre les échantillons à détecter, le milieu de culture contenant la culture, le filtre de canalisation en particulier pour collecter les bactéries et la tête de filtre dans ladite cabine de stérilisation, fermer les mécanismes de séparation de cabine des deux côtés de la cabine de stérilisation et réaliser la stérilisation, ensuite ventiler pour équilibrer la pression dans la cabine de stérilisation après avoir fini la stérilisation ;
la troisième étape : ouvrir le mécanisme de séparation de cabine entre la cabine de stérilisation et la cabine de retrait d'emballage central, transmettre les échantillons à détecter, le milieu de culture contenant la culture, le filtre de canalisation en particulier pour collecter des bactéries et la tête de filtre dans la cabine de retrait d'emballage central, fermer les mécanismes de séparation de cabine des deux côtés de la cabine de retrait d'emballage central, et retirer les emballages centraux du filtre et de la tête de filtre ;
la quatrième étape : ouvrir le mécanisme de séparation de cabine entre la cabine de retrait d'emballage central et la cabine de retrait d'emballage interne, transmettre les échantillons à détecter, le milieu de culture contenant la culture, le filtre de canalisation en particulier pour collecter les bactéries et la tête de filtre dans la cabine de retrait d'emballage interne, fermer les mécanismes de séparation de cabine des deux côtés de la cabine de retrait d'emballage interne, et retirer l'emballage interne du filtre et de la tête de filtre ;
la cinquième étape : ouvrir le mécanisme de séparation de cabine entre la cabine de retrait d'emballage interne et la cabine de commande d'enrichissement, transmettre les échantillons à détecter, le milieu de culture contenant la culture, le filtre de canalisation en particulier pour collecter les bactéries et la tête de filtre dans la cabine de commande d'enrichissement, fermer les mécanismes de séparation de cabine des deux côtés de la cabine de commande d'enrichissement, filtrer et enrichir les microorganismes contenus dans les échantillons à détecter dans le filtre, entourer le fond de filtre par la tête de filtre, injecter sélectivement ensuite la culture dans le filtre, placer le filtre enrichi et entouré sur l'appareil de transmission, obtenir un nombre approprié de filtres enrichis et entourés ;
la sixième étape : ouvrir le mécanisme de séparation de cabine entre la cabine de commande d'enrichissement et la cabine tampon, transmettre les filtres enrichis et entourés dans la cabine tampon, fermer les mécanismes de séparation de cabine des deux côtés de la cabine tampon, couper la canalisation de filtres enrichis et entourés ; et
la septième étape : ouvrir le mécanisme de séparation de cabine entre la cabine tampon et la cabine de remplissage de bactéries positives, transmettre les filtres enrichis et entourés dans la cabine de remplissage de bactéries positives, fermer les mécanismes de séparation de cabine des deux côtés de la cabine de remplissage de bactéries positives, verser les bactéries positives dans les filtres enrichis et entourés après l'achèvement de la coupe de canalisation, terminer ensuite l'enrichissement de microorganisme des échantillons détectés.

10. Procédé entièrement automatique d'enrichissement de microorganisme selon la revendication 9, dans lequel chaque fois que le mécanisme de séparation de cabine entre la position de fonctionnement préréglée, la cabine de stérilisation, la cabine de retrait d'emballage central, la cabine de retrait d'emballage interne et la cabine de commande d'enrichissement s'ouvrent, la pression de la première cabine est inférieure à celle de la dernière cabine, pour garantir qu'aucune bactérie ne soit prise dans la cabine de commande d'enrichissement ; et chaque fois que les mécanismes de séparation de cabine entre la cabine de commande d'enrichissement, la cabine tampon et la cabine de remplissage de bactéries positives s'ouvrent, la pression de la première cabine est supérieure à celle de la dernière cabine, pour garantir qu'aucune bactérie positive ne soit défavorablement transférée dans la cabine de commande d'enrichissement ; et/ou
dans ladite septième étape, après la fermeture des mécanismes de séparation de cabine des deux côtés de la cabine de remplissage de bactéries positives, une autre étape est comprise : stériliser la cabine tampon ; et/ou
lesdits filtres sont trois réservoirs parallèles avec des sorties aux deux extrémités, les cultures sont sélectivement injectées dans deux de ces réservoirs maximum ; et/ou
dans ladite cinquième étape, les échantillons et le milieu de culture utilisés sont à nouveau transférés dans la cabine de retrait d'emballage interne par l'appareil de transmission pour recyclage.
